# EUROPEAN PATENT APPLICATION

(11) **EP 4 772 878 A1**
(43) Date of publication of application: **08.07.2026**
(21) Application number: 25150270.4
(22) Date of filing: 04.01.2025
(51) Int. Cl.: G01N 33/50, G01N 33/68

(54) **AN ASSAY FOR IDENTIFYING A SUBJECT SUFFERING FROM A DISEASE WITH AN (AUTO)IMMUNE COMPONENT BASED ON THE DETECTION OF A CYTOKINE SIGNATURE**

(71) Applicant: Universität zu Lübeck, 23562 Lübeck (DE)
(72) Inventor: Hackel, Alexander Maximilian, 23562 Lübeck (DE); Riemekasten, Gabriela, 23562 Lübeck (DE)
(74) Representative: Richter, Carsten

(57) **Abstract**

The present invention relates to a method comprising the steps a) contacting *ex vivo* a cell from a mammalian cell line capable of secreting cytokines with a set of antibodies from a blood sample from a mammalian subject in a liquid medium and b), detecting the cytokine signature present in the liquid medium following step a). The method may be for aiding in the diagnosis of Post Covid Syndrome (PCS) in a mammalian subject comprising the steps a) contacting *ex vivo* a mammalian cell line capable of secreting cytokines with a set of antibodies from a blood sample of the mammalian subject in a liquid medium, b) detecting a cytokine signature present in the liquid medium following step a), wherein the cytokine signature detected comprises Macrophage Inflammatory Protein 1-delta (MiP_1d), Interleukin-4 (IL_4), Transforming Growth Factor Beta 3 (TGF_b_3), Placental Growth Factor (PIGF), Brain-Derived Neurotrophic Factor (BDNF), Glial Cell Line-Derived Neurotrophic Factor (GDNF), Monocyte Chemoattractant Protein 2 (MCP_2), Lymphotoxin-like Inducible Protein (LIGHT), Platelet-Derived Growth Factor BB (PDGF_BB), Hepatocyte Growth Factor (HGF) and Thrombopoietin (TPO) and the mammalian cell line is U937. The method may be for aiding in distinguishing between PCS and PCS with severe myalgic encephalomyelitis/chronic fatigue syndrome (ME/CFS) in a mammalian subject comprising the steps a) contacting *ex vivo* a mammalian cell line capable of secreting cytokines with a set of antibodies from a blood sample of the mammalian subject in a liquid medium, b) detecting a cytokine signature present in the liquid medium following step a), wherein the cytokine signature detected comprises Interleukin-1 alpha (IL_1a), Eotaxin-3 (Eotaxin_3), Transforming Growth Factor Beta 1 (TGF_b1), Growth-Regulated Oncogene Alpha (GRO_a), Interleukin-15 (IL_15), Neurotrophin 4 (NT_4), Insulin-Like Growth Factor I (IGF_I), Tumor Necrosis Factor Beta (TNF_b), Interleukin-1 beta (IL_1_b), Interleukin-5 (IL_5), Glial Cell Line-Derived Neurotrophic Factor (GDNF) and Interleukin-7 (IL_7) and the mammalian cell line is U937.

## Description

The present invention relates to a method comprising the steps a) contacting ex *vivo* a cell from a mammalian cell line capable of secreting cytokines with a set of antibodies from a blood sample from a mammalian subject in a liquid medium and b) detecting the cytokine signature present in the liquid medium following step a). The method may be for aiding in the diagnosis of Post Covid Syndrome (PCS) in a mammalian subject comprising the steps a) contacting ex *vivo* a mammalian cell line capable of secreting cytokines with a set of antibodies from a blood sample of the mammalian subject in a liquid medium, b) detecting a cytokine signature present in the liquid medium following step a), wherein the cytokine signature detected comprises Macrophage Inflammatory Protein 1-delta (MiP_1d), Interleukin-4 (IL_4), Transforming Growth Factor Beta 3 (TGF_b_3), Placental Growth Factor (PIGF), Brain-Derived Neurotrophic Factor (BDNF), Glial Cell Line-Derived Neurotrophic Factor (GDNF), Monocyte Chemoattractant Protein 2 (MCP_2), Lymphotoxin-like Inducible Protein (LIGHT), Platelet-Derived Growth Factor BB (PDGF_BB), Hepatocyte Growth Factor (HGF) and Thrombopoietin (TPO) and the mammalian cell line is U937. The method may be for aiding in distinguishing between PCS and PCS with severe myalgia encephalomyelitis/chronic fatigue syndrome (ME/CFS) in a mammalian subject comprising the steps a) contacting ex *vivo* a mammalian cell line capable of secreting cytokines with a set of antibodies from a blood sample of the mammalian subject in a liquid medium, b) detecting a cytokine signature present in the liquid medium following step a), wherein the cytokine signature detected comprises Interleukin-1 alpha (IL_1a), Eotaxin-3 (Eotaxin_3), Transforming Growth Factor Beta 1 (TGF_b1), Growth-Regulated Oncogene Alpha (GRO_a), Interleukin-15 (IL_15), Neurotrophin 4 (NT_4), Insulin-Like Growth Factor I (IGF _I), Tumor Necrosis Factor Beta (TNF_b), lnterleukin-1 beta (IL_1_b), Interleukin-5 (IL_5), Glial Cell Line-Derived Neurotrophic Factor (GDNF) and Interleukin-7 (IL_7) and the mammalian cell line is U937.

Autoantibodies are antibodies directed to structures of the body of the subject that produces them. Their presence may be associated with a specific disease, often directly or indirectly linked with an autoimmune response. For example, islet cell autoantibodies, antibodies to insulin, glutamic acid decarboxylase and protein tyrosine phosphatase have been described as a hallmark of diabetes type I (LeRoith, D, Taylor, S. I. Olefsky, J. M., Diabetes mellitus - a fundamental and clinical text, 3rd edition, Lippincott, Williams & Wilkins, 2004). In other cases, a disease may be associated with the absence of one or more autoantibodies. For example, the anti-DFS70 antibody has been widely used as a marker to exclude that a subject has systemic autoimmune rheumatic diseases (Duran AC, Cuzdan N, Atik TK. The clinical significance of anti-DFS70 autoantibodies and its correlation with Vitamin D levels. North Clin Istanb. 2022 Nov 16;9(6):581-589. doi: 10.14744/nci.2021.22800. PMID: 36685631; PMCID: PMC9833381.).

The fact that the presence (or absence) of autoantibodies correlates with specific diseases may be exploited for diagnostic purposes. It should be borne in mind that early diagnosis and treatment of autoimmune diseases is important because any delay can result in rapid progression of the disease and irreversible damage. By contrast, early and appropriate treatment, usually administrating some form of immunosuppressive therapy, may reverse some of the damage. In some cases, a complete recovery is possible. For example, 80% of patients suffering from NMDA receptor encephalitis have a good outcome following immunosuppressive treatment, although long-term mental problems or a recurrence are possible.

If a disease-specific autoantibody can be detected in a sample from a subject which comprises a set of representative autoantibodies, this indicates that the patient is more likely to suffer from the autoantibody-associated disease than an average subject the samples of which do not contain the autoantibody.

In some cases, the detection of a disease-specific autoantibody is an exceptionally powerful diagnostic tool. For example, an autoantibody to NMDAR may be detected to determine whether or not whether a patient suffers from anti-NMDA receptor encephalitis with a sensitivity of almost 90% (Kaneko A, Kaneko J, Tominaga N, Kanazawa N, Hattori K, Ugawa Y, Moriya A, Kuzume D, Ishima D, Kitamura E, Nishiyama K, lizuka T. Pitfalls in clinical diagnosis of anti-NMDA receptor encephalitis. J Neurol. 2018 Mar;265(3):586-596. doi: 10.1007/s00415-018-8749-3. Epub 2018 Jan 22. PMID: 29356973), while previously only a combination of unspecific parameters could be relied on as a basis for the diagnosis of the disease.

The detection of other autoantibodies as a single biomarker for a diagnosis may only have rather limited value. For example, few patients in large cohorts were shown to have autoantibodies against neurochondrin (Shelly S, Kryzer TJ, Komorowski L, Miske R, Anderson MD, Flanagan EP, Hinson SR, Lennon VA, Pittock SJ, McKeon A. Neurochondrin neurological autoimmunity. Neurol Neuroimmunol Neuroinflamm. 2019 Sep 11;6(6):e612. doi: 10.1212/NXI.0000000000000612. PMID: 31511329; PMCID: PMC6745726.). Therefore, a single-parameter diagnostic assay based on the detection of autoantibodies against neurochrondrin by itself is of limited help. By contrast, a panel of autoantibody biomarkers, each of them with a low sensitivity, but in their entirety with one that is significant, can make a valuable diagnostic contribution.

Taking into account that it is technically challenging to identify diagnostically valuable autoantibodies, frequently present only at low concentrations and in a mixture with a multitude of other antibodies, and to set up immunoassays detecting a large set of them, each indicative of a certain disease, but with a low specificity, and that health insurances are unlikely to reimburse such costs, the commercial value of such multi-parameter assays is limited and may not justify the costly regulatory approval of detection assays. As a result, many biomarkers, which, in their entirety, may well have significant value, are not exploited in the clinic.

Therefore, there is a need for novel concepts to detect complex autoimmune responses associated with diseases, for example in the form of the release of multiple antibodies against unidentified epitopes. This is particularly true if a diagnostic gap exists, for example because the molecular mechanisms underlying a disease of interest which could provide a lead for identifying a specific biomarker remain to be elucidated.

Post-COVID syndrome (PCS) is an example of such a disease. It affects 10 to 20% of the subjects recovering from mild-to-moderate COVID-19, with symptoms persisting for over six months and severely impacting daily functioning (DOI:10.1016/j.eclinm.2021.101019; doi: 10.1038/s41467-021-26513-3; doi: 10.1016/j.bbi.2021.12.020; doi: 10.1001/jamanetworkopen.2021.0830). Among these subjects, approximately half meet the Canadian Consensus Criteria (CCC) for ME/CFS, also referred to as pcME/CFS, while the remaining patients are classified as PCS (doi: 10.1038/s41467-022-32507-6; doi: 10.3390/medicina57050510; doi: 10.1300/J092v11n01_02).

The mechanisms underlying these diseases are poorly understood. Emerging evidence implicates a complex interplay of immune dysregulation, endothelial dysfunction, and vascular anomalies in PCS, which differs from these in PCS with ME/CFS (doi: 10.1016/j.ijcard.2021.10.140; doi: 10.3390/cells11152376). Autoantibodies identified in acute COVID-19 patients were among the first immunological abnormalities associated with these severe outcomes. They include antibodies to anti phospholipid and antibodies against anti-type I interferon, which drive prothrombotic states and immune imbalance ([doi: 10.1126/science.abd4585; doi: 10.1126/scitranslmed.abd3876). Additionally, functional autoantibodies targeting extracellular antigens, such as cytokines, chemokines, and vasoregulatory molecules, have been identified as significant contributors to immune dysregulation in both acute and post-viral syndromes (doi: 10.1038/s41586-021-03631-y). For example, autoantibodies against renin-angiotensin system-related proteins, including angiotensin-converting enzyme 2 and angiotensin type-1 receptor, correlate with disease severity in acute COVID-19 and vascular dysfunction in PCS (doi: 10.1016/j.jaut.2021.102683; doi: 10.10381

At the moment, there is no biomarker-based test for either PCS or for distinguishing PCS with ME/CFS from PCS. A range of tests may be carried out to exclude diseases such as anaemia, lack of thyroid gland activity or liver and kidney diseases. Symptoms associated with PCS include dyspnoea, palpitations, chest pain, fatigue, body aches, sleep disturbances, inability to concentrate and severe cognitive impairment (Sharma SK, Mohan A, Upadhyay V. Long COVID syndrome: An unfolding ertigma. Indian J Med Res. 2024 Jun;159(6):585-600. doi: 10.25259/IJMR_1449_23. PMID: 39382470; PMCID: PMC11463850.). Symptoms associated with ME/CFS include fatigue, sleep problems, issues with thinking and concentration, in particular after mental or physical activity. However, these are symptoms that are highly unspecific and difficult to assess in a standardized manner (Peo LC, Wiehler K, Paulick J, Gerrer K, Leone A, Viereck A, Haegele M, Stojanov S, Warlitz C, Augustin S, Alberer M, Hattesohl DBR, Froehlich L, Scheibenbogen C, Jason LA, Mihatsch LL, Pricoco R, Behrends U. Pediatric and adult patients with PCS with ME/CFS following COVID-19: A structured approach to diagnosis using the Munich Berlin Symptom Questionnaire (MBSQ). Eur J Pediatr. 2024 Mar;183(3):1265-1276. doi: 10.1007/s00431-023-05351-z. Epub 2023 Dec 14. PMID: 38095713; PMCID: PMC10951047). For example, fatigue is also a common symptom in diseases such as diabetes, depression and anxiety. Clearly, a simple, direct test would be a major advance in the diagnosis of PCS and PCS with ME/CFS and may become an important research tool for projects aiming to develop a treatment against PCS and/or PCS with ME/CFS.

Murthy S, Wannick M, Eleftheriadis G, Müller A, Luo J, Busch H, Dalmann A, Riemekasten G, and Sadik CD., Rheumatology (Oxford). 2021 Jun 18;60(6):3012-3022 disclose the stimulation of monocyte-like THP-1 cells by IgG and analysis of their secretome, but do not disclose a method for aiding in diagnosing a disease or for distinguishing between a first and a second disease comprising the steps determining and detecting a disease-specific cytokine signature. Kurlander, R. J. in J Immunol. 1983 Jul;131(1):140-7 discloses the binding of murine antibodies, but not human antibodies, nor autoantibodies to U937 cells, let alone determining and detecting a disease-specific cytokine signature for aiding in diagnosing a disease or distinguishing between a first and a second disease.

Therefore, the problem underlying the present invention is to provide an assay for aiding in the diagnosis of diseases characterized by a complex immune response, in particular multiple antibodies, in particular autoantibodies, each binding to different targets, preferably on the surface of a cell.

Another problem underlying the present invention is to provide a method for developing such an assay and to provide reagents for such an assay or for the method for developing it.

Another problem underlying the present invention is to provide an assay for aiding in the diagnosis of PCS.

Another problem underlying the present invention is to provide an assay for aiding in distinguishing between PCS and PCS with ME/CFS.

In a first aspect, the problem underlying the present invention is solved by a method comprising the steps
a) contacting ex *vivo* a cell from a mammalian cell line capable of secreting cytokines in a liquid medium with a set of antibodies from a blood sample from a mammalian subject which is known to be healthy or to suffer from a first disease and
b) detecting the level of at least one cytokine present in the liquid medium following step a), and
c) contacting ex *vivo* a cell from a mammalian cell line capable of secreting cytokines with a set of antibodies from a blood sample from a mammalian reference subject which is known to be healthy or to suffer from a second disease in a liquid medium, and
d) detecting the level of the at least one cytokine present in the liquid medium following step c), and
e) determining a cytokine signature by determining the difference between the level of the at least one cytokine determined in step b) and the level of the at least one cytokine determined in step d).

Such a method may be a method for determining a cytokine signature for aiding in detecting or diagnosing a disease if the sample in a) is from a subject known to suffer from the disease of interest and the sample in c) is known not to suffer from said diseases, preferably is known to be healthy. Such a method may be a method for determining a cytokine signature for aiding in distinguishing between a first disease and a second disease if the sample in a) is from a subject known to suffer from the first disease and the subject in c) is known to suffer from the second disease.

In a preferred embodiment, the method is carried out using a sample from at least one subject suffering from a disease in step a) and using a sample from at least one subject who does not suffer from said disease in step c). In a preferred embodiment, the cytokine signature defined in step e) may then be used to aid in diagnosing the disease.

In a preferred embodiment, the method is carried out using a sample from at least one subject suffering from a first disease in step a) and using a sample from at least one subject who suffers from a second disease in step c). In an embodiment, the cytokine signature defined in step e) may then be used to aid in distinguishing between the first and the second disease.

In a second aspect, the problem underlying the present invention is solved by a method for aiding in diagnosing whether a mammalian subject is likely to suffer from a disease comprising the steps
a) contacting *ex vivo* a mammalian cell line capable of secreting cytokines with a set of antibodies from a blood sample of the mammalian subject in a liquid medium,
b) detecting the presence of a cytokine signature in the liquid medium following step a), wherein the cytokine signature is a disease-specific cytokine signature which indicates that the subject is likely to suffer from the disease.
In a preferred embodiment, the cytokine signature specific for the first disease is or has been determined according to the first aspect of the invention.

In a preferred embodiment, the disease-specific cytokine signature is or has been determined according to the first aspect of the invention.

In a preferred embodiment of the second aspect, the problem underlying the present invention is solved by a method for aiding in the diagnosis of PCS in a mammalian subject comprising the steps
a) contacting *ex vivo* a mammalian cell line capable of secreting cytokines with a set of antibodies from a blood sample of the mammalian subject in a liquid medium,
b) detecting the presence of a cytokine signature in the liquid medium following step a),
wherein the cytokine signature detected comprises one or more, preferably all of the group comprising Macrophage Inflammatory Protein 1-delta (MIP_1d), Interleukin-4 (IL_4), Transforming Growth Factor Beta 3 (TGF_b_3), Placental Growth Factor (PIGF), Brain-Derived Neurotrophic Factor (BDNF), Glial Cell Line-Derived Neurotrophic Factor (GDNF), Monocyte Chemoattractant Protein 2 (MCP_2), Lymphotoxin-like Inducible Protein (LIGHT), Platelet-Derived Growth Factor BB (PDGF_BB), Hepatocyte Growth Factor (HGF) and Thrombopoietin (TPO).
and the mammalian cell line is preferably U937.

In a preferred embodiment, the cytokine signature comprises in addition one or more, preferably all from the group comprising Stem Cell Factor (SCF), Fractalkine (CX3CL1), Fibroblast Growth Factor 9 (FGF_9), Macrophage-Derived Chemokine (MDC), Monocyte Chemoattractant Protein 4 (MCP_4), Fibroblast Growth Factor 6 (FGF_6), Interleukin-7 (IL_7), Interleukin-5 (IL_5), Leukemia Inhibitory Factor (LIF), and Oncostatin M (Oncostatin_M).

In a third aspect, the problem underlying the present invention is solved by a method for aiding in distinguishing whether a mammalian subject is likely to suffer from a first disease or a second disease comprising the steps
a) contacting ex *vivo* a mammalian cell line capable of secreting cytokines with a set of antibodies from a blood sample of the mammalian subject in a liquid medium,
b) detecting the presence of a cytokine signature in the liquid medium following step a), wherein the cytokine signature is a cytokine signature specific for the first disease and its presence indicates that the subject is likely to suffer from the first disease rather than from the second disease.

In a preferred embodiment, the cytokine signature specific for the first disease is or has been determined according to the first aspect of the invention.

In a preferred embodiment of the third aspect, the problem underlying the present invention is solved by a method for aiding in distinguishing between PCS and PCS with ME/CFS in a mammalian subject comprising the steps
a) contacting ex *vivo* a mammalian cell line capable of secreting cytokines with a set of antibodies from a blood sample of the mammalian subject in a liquid medium,
b) detecting a cytokine signature present in the liquid medium following step a),
wherein the cytokine signature detected comprises one ore more, preferably all from the group comprising Interleukin-1 alpha (IL_1a), Eotaxin-3 (Eotaxin_3), Transforming Growth Factor Beta 1 (TGF_b1), Growth-Regulated Oncogene Alpha (GRO_a), Interleukin-15 (IL_15), Neurotrophin 4 (NT_4), Insulin-Like Growth Factor I (IGF_I), Tumor Necrosis Factor Beta (TNF_b), Interleukin-1 beta (IL_1_b), Interleukin-5 (IL_5), Glial Cell Line-Derived Neurotrophic Factor (GDNF) and Interleukin-7 (IL_7)
and the mammalian cell line is preferably U937.

In a preferred embodiment, the cytokine signature comprises in addition one or more, preferably all from the group comprising Interleukin-12 p70 (!L_12_p70), Eotaxin (Eotaxin), Insulin-Like Growth Factor Binding Protein 4 (IGFBP_4), Granulocyte-Macrophage Colony-Stimulating Factor (GM_CSF) and Monocyte Chemoattractant Protein 4 (MCP_4).

In a preferred embodiment, the set of antibodies comprises isolated and/or concentrated antibodies from the sample from the subject.

In a preferred embodiment, the set of antibodies comprises IgG, IgM and IgA class antibodies.

In a preferred embodiment, the set of antibodies comprises IgG class antibodies, preferably enriched relative to other classes of antibodies.

In a preferred embodiment, the set of IgG class antibodies comprises IgG class antibodies isolated from the blood sample using affinity chromatography.

In a preferred embodiment, the method comprises the step separating the cell from the liquid medium following step b).

In a preferred embodiment, the liquid medium is frozen after step a).

In a preferred embodiment, a method from the group comprising ELISA, flow cytometry and antibody microarray is used to detect the cytokine signature.

In a preferred embodiment, the subject is selected from the group comprising a rodent, a dog, a bird, a cat, a horse, a sheep, a rabbit, a goat and a primate, and preferably is a human subject.

In a preferred embodiment, at least two sets of antibodies are used in separate runs, preferably one from a healthy subject and one from a subject suspected of having a disease.

In a preferred embodiment, the sample is a blood sample selected from the group comprising serum, whole blood, plasma and capillary blood.

In a preferred embodiment, the cell is an isolated cell.

The present invention is based on the surprising finding that contacting a cell line capable of secreting cytokines with a sample from a patient suffering from a disease related to the presence of antibodies, in particular autoantibodies leads to the release of a cytokine signature into a liquid medium used to culture the cell which is distinct and characteristic for said disease. The inventors theorize that the cell releases such specific cytokine signature in response to the binding of certain antibodies. Said cytokine signature may be detected to aid in diagnosing a patient suffering from the disease if a sample from the patient comprising a representative set of their antibodies, in particular autoantibodies, is present in the sample used to contact the cell line. Said cytokine signature may also be detected to aid in distinguishing a first disease and a second disease from each other. The latter method is particularly useful if such two diseases are difficult to distinguish using state of the art methods. Of note, the methods provided according to the present invention do not rely on the direct detection antibodies such as autoantibodies in the sample of the patient, but on the detection of a cellular response by a cell in an artificial environment which cell releases a characteristic cytokine signature modulated by the presence of such antibodies. This cellular response may be triggered by complex sets of antibodies, potentially at low concentrations, the detection of which may be beyond technical feasibility using conventional methods.

Furthermore, the present invention is based on the surprising finding that contacting a cell line capable of secreting cytokines with a sample from a patient suffering from PCS triggers the release of a cytokine signature characteristic of PCS into a liquid medium. Detecting a cytokines signature comprising Macrophage Inflammatory Protein 1-delta (MIP_1d), Interleukin-4 (IL_4), Transforming Growth Factor Beta 3 (TGF_b_3), Placental Growth Factor (PIGF), Brain-Derived Neurotrophic Factor (BDNF), Glial Cell Line-Derived Neurotrophic Factor (GDNF), Monocyte Chemoattractant Protein 2 (MCP_2), Lymphotoxin-like Inducible Protein (LIGHT), Platelet-Derived Growth Factor BB (PDGF_BB), Hepatocyte Growth Factor (HGF) and Thrombopoietin (TPO) is a preferred option to detect the release of said cytokine signature characteristic of PCS.

Furthermore, the present invention is based on the surprising finding that contacting a cell line capable of secreting cytokines with a sample from a patient suffering from PCS triggers the release of two distinct cytokine signatures into a liquid medium, depending on whether the patient suffers from PCS or PCS with ME/CFS. Detecting a cytokines signature comprising lnterleukin-1 alpha (IL_1a), Eotaxin-3 (Eotaxin_3), Transforming Growth Factor Beta 1 (TGF_b1), Growth-Regulated Oncogene Alpha (GRO_a), Interleukin-15 (IL_15), Neurotrophin 4 (NT_4), Insulin-Like Growth Factor I (lGF_I), Tumor Necrosis Factor Beta (TNF_b), Interleukin-1 beta (IL_1_b), Interleukin-5 (IL_5), Glial Cell Line-Derived Neurotrophic Factor (GDNF) and Interleukin-7 (IL_7) is a preferred option to determine whether a cytokine signature characteristic of PCS or a cytokine signature characteristic of PCS with severe ME/CFS has been released.

In a preferred embodiment, the term "cytokine signature", as used herein, refers to at least one cytokine threshold level. The threshold may be a minimum threshold level, i.e. the cytokine level is considered elevated if it is higher than the threshold. The threshold may be a maximum threshold, i.e. the cytokine level is considered lowered if it is lower than the threshold. A level of the cytokine detected in the liquid medium after contacting the cell with a sample which is higher than the threshold value, i.e. a minimum threshold, may be considered an elevated level. For example, an elevated level of a specific cytokine may indicate that the subject is more likely to suffer from a disease relative to a reference subject. In another example, a lower level of a specific cytokine in the sample of a subject, i.e. below the maximum level, may indicate that the subject is more likely to suffer from a disease relative to a reference subject. Not all cytokines of the cytokine signature need either be elevated or lowered. While an elevated level of one cytokine which is part of the cytokine signature may indicate that the subject is more likely to suffer from a disease relative to a reference subject, the same may hold true for a lowered level of another cytokine in the same cytokine signature.

In a preferred embodiment, the term "determining a cytokine signature", as used herein, refers to the identification of a group of cytokines which may be used to aiding in the diagnosis of a disease or in distinguishing between a first disease and a second disease according to the present invention. This may involve carrying out the method according to the first aspect of the invention and determining for each of a number of cytokines whether or not their level in the liquid medium differs significantly and specifically depending on whether a sample from a subject suffering from a disease or a first disease or a sample from a healthy subject or a subject suffering from a second disease, respectively, is used to contact the cell. This may be determined for a range of cytokines, and the result will be that the levels of some of them do not differ significantly and specifically, wherein the levels of others may be elevated or lowered depending on whether the subject is healthy or suffers from the disease or whether the subject suffers from the first or the second disease. A sufficiently large group of samples from healthy subjects and from subjects suffering from the disease have to be compared to obtain statistically significant results for aiding in the diagnosis of the disease. Likewise, a sufficiently large group of samples from subjects suffering from the first disease and from subjects suffering from the second disease have to be compared to obtain statistically significant results for aiding in distinguishing the first disease from the second disease.

Once a cytokine signature in a sample characteristic for a disease has been determined, it may be used to aid in the diagnosis of the disease according to the second aspect of the present invention. In a preferred embodiment, the term "detecting a cytokine signature", as used herein, means that the level of each of the cytokines of the cytokine signature in the liquid medium has to be determined. Subsequently, each level has to be compared with its threshold in the signature. The cytokine signature is detected if all cytokine values are above their minimum thresholds or below their maximum thresholds, respectively. For example, if the cytokine signature comprises two cytokines A and B which are elevated if a sample from a subject suffering from the disease is examined in the method according to the second aspect of the invention and one cytokine C which is lowered if a sample from a subject suffering from the disease is examined in the method according to the second aspect of the invention, the cytokine signature is detected and the subject is likely to suffer from the disease if A and B are elevated, i.e. higher than their minimum threshold levels, and C is lowered, i.e. below its maximum threshold level. By contrast, if the cytokine signature comprises two cytokines A and B which are elevated if a sample from a subject suffering from the disease is examined in the method according to the second aspect of the invention and one cytokine C which is lowered if a sample from a subject suffering from the disease is examined in the method according to the second aspect of the invention, the cytokine signature is not detected and the subject is not likely to suffer from the disease if A, but not B is elevated and C is lowered.

Once a cytokine signature in a sample characteristic for a first disease rather than for a second disease to be distinguished from the first disease has been determined, it may be used to aid in distinguishing between a first and a second disease according to the third aspect of the present invention. In a preferred embodiment, the term "detecting a cytokine signature", as used herein, means that the level of each of the cytokines of the cytokine signature in the liquid medium has to be determined. Subsequently, each level has to be compared with its threshold in the signature. The cytokine signature is detected if all cytokine values are above their minimum thresholds or below their maximum thresholds, respectively. For example, if the cytokine signature comprises two cytokines A and B which are elevated if a sample from a subject suffering from the first disease is examined in the method according to the third aspect of the invention and one cytokine C which is lowered if a sample from a subject suffering from the first disease is examined in the method according to the third aspect of the invention, the cytokine signature is detected and the subject is likely to suffer from the first disease rather than from the second disease if A and B are elevated, i.e. higher than their minimum threshold levels, and C is lowered, i.e. below its maximum threshold level. By contrast, if the cytokine signature comprises two cytokines A and B which are elevated if a sample from a subject suffering from the first disease is examined in the method according to the third aspect of the invention and one cytokine C which is lowered if a sample from a subject suffering from the disease is examined in the method according to the second aspect of the invention, the cytokine signature is not detected and the subject is not likely to suffer from the first disease if A, but not B is elevated and C is lowered.

In a preferred embodiment, the cytokine signature comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75 or 80 cytokines. In a preferred embodiment, the cytokine signature comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 35, at least 40, at least 45, at least 50, at least 55, at least 60, at least 65, at least 70, at least 75 or at least 80 cytokines.

Typically, one specific cytokine may be insufficient to determine a cytokine signature that is the basis for a statistically significant diagnostic result, but as the number of cytokines included in the cytokine signature is increased, the diagnostic reliability, in particular the sensitivity and the specificity of the overall method, are improved. For example, a cytokine signature determined comprising ten cytokines may provide a more diagnostically sound result, i.e. better specificity and/or sensitivity, than one determined with a cytokine signature comprising one or two cytokines only. In a preferred embodiment, the level of each cytokine must be determined. The threshold value which indicates whether a cytokine level is increased or decreased and whether a subject is likely to suffer from a disease may be determined by comparing for each cytokine of the cytokine signature sufficiently large cohorts of patients, one cohort with samples from healthy subjects and one cohort with samples from subjects suffering from the disease of interest. In another embodiment, the threshold value which indicates whether a cytokine level is increased or decreased and whether a subject is likely to suffer from a first disease rather than from a second disease may be determined by comparing for each cytokine of the cytokine signature sufficiently large cohorts of patients, one cohort with samples from subjects suffering from the first disease and one cohort with samples from subjects suffering from the second disease. The person skilled in the art is familiar with methods that may be used to calculate threshold levels, and they are described in the state of the art, for example in Huang, R.P., Burkholder, B., Jones, V. S., Jiang, W.D., Mao, Y. Q., Chen, Q. L., and Shi, Z. (2012) Cytokine Arrays in Biomarker Discovery and Validation, Current Proteomics 9(1), 55-70 and Amaratunga, D., and Cabrera, J., Exploration and Analysis of DNA Microarray and Protein Array Data, Wiley Interscience, 2004.

In a preferred embodiment, the cytokine signature is determined such that the sensitivity of the assay is at least 60%. In a preferred embodiment, the cytokine signature is chosen such that the sensitivity of the assay is at least 65%. In a preferred embodiment, the cytokine signature is chosen such that the sensitivity of the assay is at least 70%. In a preferred embodiment, the cytokine signature is chosen such that the sensitivity of the assay is at least 75%. In a preferred embodiment, the cytokine signature is chosen such that the sensitivity of the assay is at least 80%. In a preferred embodiment, the cytokine signature is chosen such that the sensitivity of the assay is at least 85%. In a preferred embodiment, the cytokine signature is chosen such that the sensitivity of the assay is at least 90%. In a preferred embodiment, the cytokine signature is chosen such that the sensitivity of the assay is at least 95%. In a preferred embodiment, the cytokine signature is chosen such that the sensitivity of the assay is at least 97%. In a preferred embodiment, the cytokine signature is chosen such that the sensitivity of the assay is at least 99%. In a preferred embodiment, the cytokine signature is chosen such that the sensitivity of the assay is at least 99.5%.

In a preferred embodiment, the cytokine signature is determined such that the specificity of the assay is at least 60%. In a preferred embodiment, the cytokine signature is chosen such that the specificity of the assay is at least 65%. In a preferred embodiment, the cytokine signature is chosen such that the specificity of the assay is at least 70%. In a preferred embodiment, the cytokine signature is chosen such that the specificity of the assay is at least 75%. In a preferred embodiment, the cytokine signature is chosen such that the specificity of the assay is at least 80%. In a preferred embodiment, the cytokine signature is chosen such that the specificity of the assay is at least 85%. In a preferred embodiment, the cytokine signature is chosen such that the specificity of the assay is at least 90%. In a preferred embodiment, the cytokine signature is chosen such that the specificity of the assay is at least 95%. In a preferred embodiment, the cytokine signature is chosen such that the specificity of the assay is at least 97%. In a preferred embodiment, the cytokine signature is chosen such that the specificity of the assay is at least 99%. In a preferred embodiment, the cytokine signature is chosen such that the specificity of the assay is at least 99.5%.

In a preferred embodiment, the cytokine signature is determined such that the specificity and the sensitivity are each 60% or more. In a preferred embodiment, the cytokine signature is determined such that the specificity and the sensitivity are each 70% or more. In a preferred embodiment, the cytokine signature is determined such that the specificity and the sensitivity are each 75% or more. In a preferred embodiment, the cytokine signature is determined such that the specificity and the sensitivity are each 80% or more. In a preferred embodiment, the cytokine signature is determined such that the specificity and the sensitivity are each 82.5% or more. In a preferred embodiment, the cytokine signature is determined such that the specificity and the sensitivity are each 85% or more. In a preferred embodiment, the cytokine signature is determined such that the specificity and the sensitivity are each 87.5% or more. In a preferred embodiment, the cytokine signature is determined such that the specificity and the sensitivity are each 90% or more. In a preferred embodiment, the cytokine signature is determined such that the specificity and the sensitivity are each 92.5% or more. In a preferred embodiment, the cytokine signature is determined such that the specificity and the sensitivity are each 95% or more. In a preferred embodiment, the cytokine signature is determined such that the specificity and the sensitivity are each 96% or more. In a preferred embodiment, the cytokine signature is determined such that the specificity and the sensitivity are each 97% or more. In a preferred embodiment, the cytokine signature is determined such that the specificity and the sensitivity are each 98% or more. In a preferred embodiment, the cytokine signature is determined such that the specificity and the sensitivity are each 99% or more. In a preferred embodiment, the cytokine signature is determined such that the specificity and the sensitivity are each 99.5% or more.

Methods that may be used to detect the concentration of a cytokine and thus to determine or to detect a cytokine signature may be selected from the group comprising ELISA, flow cytometry and antibody microarray have been described in the state of the art, for example in Chiswick, E.L., et al., Detection and Quantification of Cytokines and Other Biomarkers. In: Ashman, R. (eds) Leucocytes. Methods in Molecular Biology, vol 844. Humana Press. 2012. https://doi.org/10.1007/978-1-61779-527-5_2Conn, P. M., Handbook of Proteomic Methods, Springer Science, 2003, or in Thomson, A. W., and Lotze, M. T., The Cytokine Handbook, 4th edition, Volume 1, Academic press, 2003. An antibody array based on fluorescence detection (e.g. RayBiotech, Norcross, GA, USA, #AAH-CYT-G5-8) is a preferred option.

In a preferred embodiment, the at least one cytokine in the cytokine signature, preferably more than one cytokine is selected from the group comprising Macrophage Inflammatory Protein 1-delta (MIP_1d), Interleukin-4 (IL_4), Transforming Growth Factor Beta 3 (TGF_b_3), Placental Growth Factor (PIGF), Brain-Derived Neurotrophic Factor (BDNF), Glial Cell Line-Derived Neurotrophic Factor (GDNF), Monocyte Chemoattractant Protein 2 (MCP_2), Lymphotoxin-like Inducible Protein (LIGHT), Platelet-Derived Growth Factor BB (PDGF_BB), Hepatocyte Growth Factor (HGF) and Thrombopoietin (TPO), Interleukin-1 alpha (IL_1a), Eotaxin-3 (Eotaxin_3), Transforming Growth Factor Beta 1 (TGF_b1), Growth-Regulated Oncogene Alpha (GRO_a), Interleukin-15 (IL_15), Neurotrophin 4 (NT_4), Insulin-Like Growth Factor I (IGF_I), Tumor Necrosis Factor Beta (TNF_b), Interleukin-1 beta (IL_1_b), Interleukin-5 (IL_5), Glial Cell Line-Derived Neurotrophic Factor (GDNF) and Interleukin-7 (IL_7), Stem Cell Factor (SCF), Fractalkine (CX3CL1), Fibroblast Growth Factor 9 (FGF_9), Macrophage-Derived Chemokine (MDC), Monocyte Chemoattractant Protein 4 (MCP_4), Fibroblast Growth Factor 6 (FGF_6), Interleukin-7 (IL_7), lnterleukin-5 (IL_5), Leukemia Inhibitory Factor (LIF), and Oncostatin M (Oncostatin_M), Interleukin-12 p70 (IL_12_p70), Eotaxin (Eotaxin), Insulin-Like Growth Factor Binding Protein 4 (IGFBP_4) and Granulocyte-Macrophage Colony-Stimulating Factor (GM_CSF).

In a preferred embodiment, Macrophage Inflammatory Protein 1-delta (MIP_1d) is represented by UniProt accession number P13236. In a preferred embodiment, Interleukin-4 (IL_4) is represented by UniProt accession number P05112. In a preferred embodiment, Transforming Growth Factor Beta 3 (TGF_b_3) is represented by UniProt accession number P10600. In a preferred embodiment, Placental Growth Factor (PIGF) is represented by UniProt accession number P49763. In a preferred embodiment, Brain-Derived Neurotrophic Factor (BDNF) is represented by UniProt accession number P23560. In a preferred embodiment, Glial Cell Line-Derived Neurotrophic Factor (GDNF) is represented by UniProt accession number P39905. In a preferred embodiment, Monocyte Chemoattractant Protein 2 (MCP_2) is represented by UniProt accession number P80075. In a preferred embodiment, Lymphotoxin-like Inducible Protein (LIGHT) is represented by UniProt accession number O43557. In a preferred embodiment, Platelet-Derived Growth Factor BB (PDGF_BB) is represented by UniProt accession number P01127. In a preferred embodiment, Hepatocyte Growth Factor (HGF) is represented by UniProt accession number P14210. In a preferred embodiment, Thrombopoietin (TPO) is represented by UniProt accession number P40225. In a preferred embodiment, Interleukin-1 alpha (IL_1a) is represented by UniProt accession number P01583. In a preferred embodiment, Eotaxin-3 (Eotaxin_3) is represented by UniProt accession number Q9Y258. In a preferred embodiment, Transforming Growth Factor Beta 1 (TGF_b1) is represented by UniProt accession number P01137. In a preferred embodiment, Growth-Regulated Oncogene Alpha (GRO_a) is represented by UniProt accession number P09341. In a preferred embodiment, Interleukin-15 (IL_15) is represented by UniProt accession number P40933. In a preferred embodiment, Neurotrophin 4 (NT_4) is represented by UniProt accession number P34130. In a preferred embodiment, Insulin-Like Growth Factor I (IGF_I) is represented by UniProt accession number P05019. In a preferred embodiment, Tumor Necrosis Factor Beta (TNF_b) is represented by UniProt accession number P01374. In a preferred embodiment, lnterleukin-1 beta (IL_1_b) is represented by UniProt accession number P01584. In a preferred embodiment, Interleukin-5 (IL_5) is represented by UniProt accession number P05113. In a preferred embodiment, Interleukin-7 (IL_7) is represented by UniProt accession number P13232. In a preferred embodiment, Stem Cell Factor (SCF) is represented by UniProt accession number P21583. In a preferred embodiment, Fractalkine (CX3CL1) is represented by UniProt accession number P78423. In a preferred embodiment, Fibroblast Growth Factor 9 (FGF_9) is represented by UniProt accession number P31371. In a preferred embodiment, Macrophage-Derived Chemokine (MDC) is represented by UniProt accession number Q99731. In a preferred embodiment, Monocyte Chemoattractant Protein 4 (MCP_4) is represented by UniProt accession number Q99616. In a preferred embodiment, Fibroblast Growth Factor 6 (FGF_6) is represented by UniProt accession number P10767. In a preferred embodiment, Leukemia Inhibitory Factor (LIF) is represented by UniProt accession number P15018. In a preferred embodiment, Oncostatin M (Oncostatin_M) is represented by UniProt accession number P13725. In a preferred embodiment, Interleukin-12 p70 (IL_12_p70) is represented by UniProt accession number A1Z2L5. In a preferred embodiment, Macrophage Inflammatory Eotaxin (Eotaxin) is represented by UniProt accession number P51671. In a preferred embodiment, Insulin-Like Growth Factor Binding Protein 4 (IGFBP_4) is represented by UniProt accession number P22692. In a preferred embodiment, Granulocyte-Macrophage Colony-Stimulating Factor (GM_CSF) is represented by UniProt accession number P04141.

In a preferred embodiment, an elevated level of Macrophage Inflammatory Protein 1-delta (MIP_1d) as part of the cytokine signature indicates an increased likelihood that a subject is not healthy, but suffers from PCS (including PCS with ME/CFS). In a preferred embodiment, an elevated level of Interleukin-4 (IL_4) as part of the cytokine signature indicates an increased likelihood that a subject is not healthy, but suffers from PCS (including PCS with ME/CFS). In a preferred embodiment, an elevated level of Transforming Growth Factor Beta 3 (TGF_b_3) as part of the cytokine signature indicates an increased likelihood that a subject is not healthy, but suffers from PCS (including PCS with ME/CFS). In a preferred embodiment, an elevated level of Placental Growth Factor (PIGF) as part of the cytokine signature indicates an increased likelihood that a subject is not healthy, but suffers from PCS (including PCS with ME/CFS). In a preferred embodiment, an elevated level of Brain-Derived Neurotrophic Factor (BDNF) as part of the cytokine signature indicates an increased likelihood that a subject is not healthy, but suffers from PCS (including PCS with ME/CFS). In a preferred embodiment, an elevated level of Glial Cell Line-Derived Neurotrophic Factor (GDNF) as part of the cytokine signature indicates an increased likelihood that a subject is not healthy, but suffers from PCS (including PCS with ME/CFS). In a preferred embodiment, an elevated level of Monocyte Chemoattractant Protein 2 (MCP_2) as part of the cytokine signature indicates an increased likelihood that a subject is not healthy, but suffers from PCS (including PCS with ME/CFS). In a preferred embodiment, an elevated level of Lymphotoxin-like Inducible Protein (LIGHT) as part of the cytokine signature indicates an increased likelihood that a subject is not healthy, but suffers from PCS (including PCS with ME/CFS). In a preferred embodiment, an elevated level of Platelet-Derived Growth Factor BB (PDGF_BB) as part of the cytokine signature indicates an increased likelihood that a subject is not healthy, but suffers from PCS (including PCS with ME/CFS). In a preferred embodiment, an elevated level of Hepatocyte Growth Factor (HGF) as part of the cytokine signature indicates an increased likelihood that a subject is not healthy, but suffers from PCS (including PCS with ME/CFS). In a preferred embodiment, an elevated level of Thrombopoietin (TPO) as part of the cytokine signature indicates an increased likelihood that a subject is not healthy, but suffers from PCS (including PCS with ME/CFS). In a preferred embodiment, an elevated level of Stem Cell Factor (SCF) as part of the cytokine signature indicates an increased likelihood that a subject is not healthy, but suffers from PCS (including PCS with ME/CFS). In a preferred embodiment, an elevated level of Fractalkine (CX3CL1) as part of the cytokine signature indicates an increased likelihood that a subject is not healthy, but suffers from PCS (including PCS with ME/CFS). In a preferred embodiment, an elevated level of Fibroblast Growth Factor 9 (FGF_9) as part of the cytokine signature indicates an increased likelihood that a subject is not healthy, but suffers from PCS (including PCS with ME/CFS). In a preferred embodiment, an elevated level of Macrophage-Derived Chemokine (MDC) as part of the cytokine signature indicates an increased likelihood that a subject is not healthy, but suffers from PCS (including PCS with ME/CFS). In a preferred embodiment, an elevated level of Monocyte Chemoattractant Protein 4 (MCP_4) as part of the cytokine signature indicates an increased likelihood that a subject is not healthy, but suffers from PCS (including PCS with ME/CFS). In a preferred embodiment, an elevated level of Fibroblast Growth Factor 6 (FGF_6) as part of the cytokine signature indicates an increased likelihood that a subject is not healthy, but suffers from PCS (including PCS with ME/CFS). In a preferred embodiment, a lowered level of Interleukin-7 (IL_7) as part of the cytokine signature indicates an increased likelihood that a subject is not healthy, but suffers from PCS (including PCS with ME/CFS). In a preferred embodiment, a lowered level of Interleukin-5 (IL_5) as part of the cytokine signature indicates an increased likelihood that a subject is not healthy, but suffers from PCS (including PCS with ME/CFS). In a preferred embodiment, an elevated level of Leukemia Inhibitory Factor (LlF) as part of the cytokine signature indicates an increased likelihood that a subject is not healthy, but suffers from PCS (including PCS with ME/CFS). In a preferred embodiment, an elevated level of Oncostatin M (Oncostatin_M) as part of the cytokine signature indicates an increased likelihood that a subject is not healthy, but suffers from PCS (including PCS with ME/CFS).

In a preferred embodiment, an elevated level of Interleukin-1 alpha (IL_1a) as part of the cytokine signature indicates an increased likelihood that a subject suffers from PCS with ME/CFS rather than PCS without ME/CFS. In a preferred embodiment, an elevated level of Eotaxin-3 (Eotaxin_3) as part of the cytokine signature indicates an increased likelihood that a subject suffers from PCS with ME/CFS rather than PCS without ME/CFS. In a preferred embodiment, an elevated level of Transforming Growth Factor Beta 1 (TGF_b1) as part of the cytokine signature indicates an increased likelihood that a subject suffers from PCS with ME/CFS rather than PCS without ME/CFS. In a preferred embodiment, an elevated level of Growth-Regulated Oncogene Alpha (GRO_a) as part of the cytokine signature indicates an increased likelihood that a subject suffers from PCS with ME/CFS rather than PCS without ME/CFS. In a preferred embodiment, an elevated level of Interleukin-15 (IL_15) as part of the cytokine signature indicates an increased likelihood that a subject suffers from PCS with ME/CFS rather than PCS without ME/CFS. In a preferred embodiment, a lowered level of Neurotrophin-4 (NT_4) as part of the cytokine signature indicates an increased likelihood that a subject suffers from PCS with ME/CFS rather than PCS without ME/CFS. In a preferred embodiment, a lowered level of Insulin-Like Growth Factor I (IGF_I) as part of the cytokine signature indicates an increased likelihood that a subject suffers from PCS with ME/CFS rather than PCS without ME/CFS. In a preferred embodiment, an elevated level of Tumor Necrosis Factor Beta (TNF_b) as part of the cytokine signature indicates an increased likelihood that a subject suffers from PCS with ME/CFS rather than PCS without ME/CFS. in a preferred embodiment, a lowered level of Interleukin-1 beta (IL_1_b) as part of the cytokine signature indicates an increased likelihood that a subject suffers from PCS with ME/CFS rather than PCS without ME/CFS. In a preferred embodiment, an elevated level of Interleukin-5 (IL_5) as part of the cytokine signature indicates an increased likelihood that a subject suffers from PCS with ME/CFS rather than PCS without ME/CFS. In a preferred embodiment, a lowered level of Glial Cell Line-Derived Neurotrophic Factor (GDNF) as part of the cytokine signature indicates an increased likelihood that a subject suffers from PCS with ME/CFS rather than PCS without ME/CFS. In a preferred embodiment, an elevated level of Interleukin-7 (IL_7) as part of the cytokine signature indicates an increased likelihood that a subject suffers from PCS with ME/CFS rather than PCS without ME/CFS.

In a preferred embodiment, a lowered level of Interleukin-12 p70 (IL_12_p70) as part of the cytokine signature indicates an increased likelihood that a subject suffers from PCS with ME/CFS rather than PCS without ME/CFS. In a preferred embodiment, a lowered level of Eotaxin (Eotaxin) as part of the cytokine signature indicates an increased likelihood that a subject suffers from PCS with ME/CFS rather than PCS without ME/CFS. In a preferred embodiment, a lowered level of Insulin-Like Growth Factor Binding Protein 4 (IGFBP_4) as part of the cytokine signature indicates an increased likelihood that a subject suffers from PCS with ME/CFS rather than PCS without ME/CFS. In a preferred embodiment, an elevated level of Granulocyte-Macrophage Colony-Stimulating Factor (GM_CSF) as part of the cytokine signature indicates an increased likelihood that a subject suffers from PCS with ME/CFS rather than PCS without ME/CFS. In a preferred embodiment, an elevated level of Monocyte Chemoattractant Protein 4 (MCP_4) as part of the cytokine signature indicates an increased likelihood that a subject suffers from PCS with ME/CFS rather than PCS without ME/CFS.

A cell which is capable of secreting cytokines upon being contacted with antibodies, in particular autoantibodies from a subject suffering from a disease with an autoimmune component is used to practice the methods according to the present invention. In a preferred embodiment, the cell is a human cell line. In a preferred embodiment, the cell is a monocyte and is more preferably from a monocytic cell line. In a preferred embodiment, the cell is a macrophage and is more preferably from a macrophage cell line. In a more preferred embodiment, the cell is from the group of cell lines comprising U937 (Sundstrom C. and Nilsson K. (1976) Establishment and characterization of a human histiocytic lymphoma cell line (U-937). International Journal of Cancer 17(5): 565-577), THP-1 (Drexler H.G., Quentmeier H. and MacLeod R.A. (2004) Malignant hematopoietic cell lines: in vitro models for the study of MLL gene alterations. Leukemia 18(2): 227-232), Mono Mac 6 (Ziegler-Heitbrock H.W., Thiel E., Futterer A., Herzog V., Wirtz A. and Riethmuller G. (1988) Establishment of a human cell line (Mono Mac 6) with characteristics of mature monocytes. International Journal of Cancer 41(3): 456-461), HL-60 (Gallagher R., Collins S., Trujillo J., McCredie K., Ahearn M., Tsai S., Metzgar R., Aulakh G., Ting R., Ruscetti F. and Gallo R. (1979) Characterization of the continuous, differentiating myeloid cell line (HL-60) from a patient with acute promyelocytic leukemia. Blood 54(3): 713-733) and J774 (Ralph P., Moore M.A. and Nilsson K. (1976) Lysozyme synthesis by established human and murine histiocytic lymphoma cell lines. Journal of Experimental Medicine 143(6): 1528-1533). These cells may be obtained from Leibniz-Institut DSMZ-Deutsche Sammlung von Mikroorganismen und Zellkulturen GmbH, for example the access code of U937 for purchase from DSMZ is ACC 5. In a preferred embodiment, any cell line refers to original cell lines, but also to derivatives modified using genetic engineering methods provided that they remain capable of secreting cytokines following contact with antibodies according to the present invention.

Step a) is carried out in a liquid medium and under conditions which enable the cell to secrete cytokines. In particular, the cell is kept in a liquid medium under conditions compatible with cellular metabolic activity, in particular the ability to release cytokines. The presence of non-viable or damaged cells which may release cytokines in an uncontrolled manner is to be avoided. The person skilled in the art is familiar with cultivating and maintaining cells and suitable media, for example Roswell Park Memorial Institute medium 1640 (RPMI 1640). Examples are described in the literature, for example in Pamies, D., et al., Guidance Document on Good Cell and Tissue Culture Practice 2.0 (GCCP 2.0) 2022, Altex-Alternatives to Animal Experimentation, 2022, https://doi.org/10.14573/altex.2111011. The liquid medium has to be essentially free of cytokines other than those secreted by the cell to make sure that background levels of cytokines or other substances do not obscure or alter the cytokine signature to be detected. Likewise, any other substances or components that may trigger the release of cytokines, accelerate their degradation, interfere with the assay used to detect cytokines or the like need to be absent. Any step contemplating that a set of antibodies is contacted with the cell line in a liquid includes an incubation long enough for the antibodies to trigger the release of a cytokine signature, for example at least 120 minutes, 4 h, 6 h, 12 h, 18 h, 24 h, preferably 24 h, at a temperature of at least 25 °C, preferably at least 30 °C, more preferably 37 °C.

In a preferred embodiment, steps a) and c) according to the first aspect may be carried out in any order, provided they are practiced under comparable conditions. Likewise, steps b) and d) may be carried out in any order under comparable conditions.

The subject is a mammalian subject, preferably a subject selected from the group comprising a rodent, a dog, a bird, a cat, a horse, a sheep, a rabbit, a goat and a primate, preferably a human subject. In a preferred embodiment, the subject is a patient suspected of suffering from a disease, preferably a disease associated with the presence of antibodies, preferably autoantibodies, more specifically antibodies, more preferably autoantibodies triggering the secretion of cytokines. In a preferred embodiment, the disease is selected from the group comprising a neurological disease, nephrological disease, metabolic disease, an endocrinological disease and cancer, each associated with the presence of at least one antibody, preferably autoantibody triggering the release of cytokines when contacted with the cell. In a preferred embodiment, the disease is a fatigue syndrome following an infection with a pathogen, preferably a virus. More preferably, the virus is selected from the group comprising Epstein-Barr and a coronavirus, preferably from the group comprising SARs-CoV-2, MERS and SARS-CoV-2, more preferably SARS-CoV-2.

In a preferred embodiment, the method is a method for aiding in the diagnosis of a disease. In a preferred embodiment, the term "diagnosis", as used herein, is to be understood in its broadest possible sense and may refer to any kind of procedure aiming to obtain information instrumental in the assessment whether a subject, known or an anonymous subject from a cohort, suffers or is likely or more likely than the average or a comparative subject, the latter preferably having similar symptoms, to suffer from a certain disease. Such information may be used for a clinical diagnosis but may also be obtained by an experimental and/or research laboratory for the purpose of general research, for example to determine the proportion of subjects suffering from the disease in a patient cohort or in a population. This includes determining whether a sample originates from a person suffering from the disease or from a healthy person, even though no treatment will be administered, for example for epidemiological research. While the result may be assigned to a specific patient for clinical diagnostic applications and may be communicated to a medical doctor or institution treating said patient, this is not necessarily the case for other applications, for example in diagnostics for research purposes, where it may be sufficient to assign the results to a sample from an anonymized subject or group of anonymized subjects. Thus, in some embodiments, the person to be diagnosed, i.e., the "subject" or "patient", is an anonymous blood donor whose blood may be donated or used to obtain therapeutically or diagnostically useful antibodies. The term "diagnosis" also refers to negative diagnosis, *i.e.,* the case where the absence of a specific cytokine signature that the patient may suffer from a disease other than a disease associated with the presence of said cytokine signature, as described herein, which may lead to the indirect diagnosis of said other disease. For example, if it is established that a subject having fatigue does not suffer from PCS including PCS with severe ME/CSF, it is more likely that they suffer from a non-autoimmune neurological or psychiatric disease such as depression or drug abuse. The person skilled in the art will appreciate that a clinician does usually not arrive at the conclusion whether or not the patient suffers or is likely to suffer from a disease, condition or disorders solely on the basis of a single diagnostic parameter, but needs to take into account other aspects, for example the presence of other antibodies, markers, blood parameters, clinical assessment of the patient's symptoms or the results of medical imaging or other non-invasive methods such as polysomnography, to arrive at a conclusive diagnosis. See Baenkler H. W. (2012), General aspects of autoimmune diagnostics, in Renz, H., Autoimmune diagnostics, 2012, de Gruyter, page 3. In a preferred embodiment, the meaning of any symptoms or diseases referred to throughout this application is in line with the person skilled in the art's understanding as of the filing date or, preferably, earliest priority date of this application as evidenced by textbooks and scientific publications. In a preferred embodiment, the methods or uses or products are not used, taken alone, to arrive at a definite, final diagnosis.

In a preferred embodiment, the method is a method for aiding in distinguishing a first and a second disease. In a preferred embodiment, the term "aiding in distinguishing a first and a second disease", as used herein, means that it is indicated if a subject suffers from a first or a second disease. Typically, this may be if a patient suffers from unspecific symptoms associated with both diseases. It may be important to distinguish between the first disease and the second disease if specific treatments are available that are in order only for one of the diseases, for example because they may be associated with side effects. For example, an immunosuppressive treatment should be applied only if a patient suffers from an autoimmune disease, since it will generally weaken the patient, whose immune response may be insufficient to fight infections following the immunosuppressive treatment. This includes determining whether the sample originates from a person suffering from the first disease or from a person suffering from the second disease, even though no treatment will be administered, for example for epidemiological research.

In a preferred embodiment, any information or data which is the result of a method according to the present invention may be communicated to the subject or a medical doctor treating the subject orally, preferably by telephone, in a written form, preferably fax or letter, or in an electronic form *via* fax or *via* the internet, for example as an email or text message.

It is understood that the diagnosis of a patient can rarely be achieved based on a single test, but that the medical history, an interview with the patient and additional parameters will have to be considered. Also, any type of diagnostic procedure is subject to occasional failures and may be useful even if the sensitivity and/or sensitivity are less than 100%. A differential diagnosis starting with basic, unspecific tests and culminating in disease-specific assays is often followed. Excluding another disease with similar symptoms may in the sense of a negative diagnosis may contribute to the diagnosis of the disease of interest. The person skilled in the art is familiar with conventional ways to diagnose PCS and PCS with ME/CFS, reviewed for example by Nacul, L., et al., European Network on Myalgic Encephalomyelitis/Chronic Fatigue Syndrome (EUROMENE): Expert Consensus on the Diagnosis, Service Provision, and Care of People with ME/CFS in Europe. Medicina (Kaunas), 2021. 57(5)). Generally, symptoms such as generalized exhaustion, cognitive dysfunction, commonly referred to as "brain fog", sleep disturbances, muscle weakness, and joint pain frequently manifest even after mild forms of SARS-CoV-2 infections. While most individuals recover within 4 to 12 weeks with appropriate rest, lifestyle adjustments, and symptom management, a subset of patients experience prolonged symptoms that persist for several months, a condition known as Post-COVID Syndrome (PCS) (Kedor, C. et al. *Nat Commun* **13**, 5104 (2022). https://doi.org/10.1038/s41467-022-32507-6). For PCS patients who do not meet the criteria for ME/CFS, therapeutic approaches focus on supporting recovery and alleviating individual symptoms. Strategies such as pacing, where physical and mental activities are carefully balanced to prevent overexertion, play a central role. Nutritional interventions, including anti-inflammatory diets rich in omega-3 fatty acids, antioxidants, and essential nutrients, are employed alongside supplements aimed at supporting mitochondrial function. In addition, pharmacological approaches targeting residual inflammation and vascular disturbances, such as low-dose corticosteroids, antihistamines, or anticoagulants, offer potential relief for some patients. Stress management, relaxation techniques, and sufficient restorative sleep remain crucial components of recovery (Barrea, L. et al. Nutrients. 2022 14(6):1305. doi: 10.3390/nu14061305). By contrast, for those PCS patients who develop ME/CFS, treatment strategies must address the multifactorial and chronic nature of the condition. Immunological therapies have emerged as a promising avenue, particularly immunoadsorption (IA), which selectively removes pathogenic autoantibodies from the bloodstream (Stein, E. et al. Efficacy of repeated immunoadsorption in patients with post-COVID myalgic encephalomyelitis/chronic fatigue syndrome and elevated β2-adrenergic receptor autoantibodies: a prospective cohort study, The Lancet Regional Health - Europe, Volume 49, 101161). Early clinical trials suggest significant symptom improvement following !A in ME/CFS patients, especially those with an autoimmune component. Similarly, intravenous immunoglobulin (IVIG) therapy has shown potential to modulate immune dysregulation and neutralize harmful autoantibodies (Graninger, M. et al. J MVir 2024. 96, 12.).

In a preferred embodiment, the term "isolated cell" as used herein, means that the cell capable of secreting cytokines is a pure cell line rather than a mixture of various cells. In a preferred embodiment, the term "pure", as used herein, means that at least 80%, 85%, 90%, 92%, 94%, 96%, 98%, 99%, 99.5% or 99.9% of the cells present belong to one cell line which is capable of releasing cytokines, guaranteed by the supplier Leibniz-lnstitut DSMZ-Deutsche Sammlung von Mikroorganismen und Zellkulturen GmbH, DSMZ._The number of cells has to be chosen such that the cytokine levels secreted into the medium are sufficient for detection. The person skilled in the art is capable of obtaining and culturing isolated cells, for example by obtaining a pure cell line from Leibniz-lnstitut DSMZ-Deutsche Sammlung von Mikroorganismen und Zellkulturen GmbH, DSMZ no.: ACC 5 and culturing them (see for example Jayakoda, T., A Beginners' Guide to Mammalian Cell Culture, Bluerose Publishers, 2022). + (Pamies, D., et al., Guidance Document on Good Cell and Tissue Culture Practice 2.0 (GCCP 2.0) 2022, Altex-Alternatives to Animal Experimentation, 2022).

In a preferred embodiment, the set of antibodies used to contact the cell comprises isolated and/or concentrated antibodies. In a preferred embodiment, the term "isolated", as used herein, means that the preparation of the antibody used comprises a lower concentration of at least one component other than the antibody or antibodies of interest compared to the original sample obtained. In a preferred embodiment, the term "concentrated", as used herein, means that the preparation of the antibody used comprises a higher concentration of the antibody or antibodies of interest compared to the original sample obtained. The person skilled in the art is familiar with methods used to isolate and/or to concentrate antibodies. For example, immunoaffinity chromatography based on immobilized antigens may be used as described in Yue X, Yin J, et al., Induced antibodies directed to the angiotensin receptor type 1 provoke skin and lung inflammation, dermal fibrosis and act species overarching. Ann Rheum Dis. 2022 Aug 11;81(9):1281-1289. Doi: 10.1136/annrheumdis-2021-222088. PMID: 35595388; PMCID: PMC9380513. And references quoted therein. The removal of components other than antibodies using immunoaffinity based on immobilized antibodies against components to be removed is described in Yue X, Yin J, et al., Induced antibodies directed to the angiotensin receptor type 1 provoke skin and lung inflammation, dermal fibrosis and act species overarching. Ann Rheum Dis. 2022 Aug 11;81(9):1281-1289. Doi: 10.1136/annrheumdis-2021-222088. PMID: 35595388; PMCID: PMC9380513. Commercial kits are available for isolating certain human antibody isotypes, for example human IgA(www.antikoerper-online, product no. ABIN6956656), human IgM (LigaTrap, product no. LT-143KIT by Dianova) or human IgG (Montage Antibody Purification Kits, product no. LSK2 ABG 20, Thermo Fisher). In a more preferred embodiment, antibodies comprising IgG, IgA and IgM are used at a purity, relative to other proteins present in the preparation of antibodies used to contact the cell, of at least 60%, 70%, 80%, 90%, 90% or 99% as judged by SDS PAGE followed by quantification of the density of visible bands. In another more preferred embodiment, IgG antibodies are used at a purity, relative to other proteins including IgA and IgM present in the preparation of antibodies used to contact the cell, of at least 60%, 70%, 80%, 90%, 90% or 99% as judged by SDS PAGE followed by quantification of the density of visible bands.

In a preferred embodiment, the cell is removed from the liquid medium following the step contacting the cell with the antibodies from the sample from the subject. This may be accomplished by spinning down the cell suspension and recovering the supernatant, preferably for further analysis, or by filtering the suspension.

In a fourth aspect of the invention, the problem underlying the present invention is solved by the use of an immunoassay capable of detecting the level of one or more, preferably all cytokines from a group of cytokines for determining a cytokine signature, wherein the group comprises Macrophage Inflammatory Protein 1-delta (MIP_1d), Interleukin-4 (IL_4), Transforming Growth Factor Beta 3 (TGF_b_3), Placental Growth Factor (PIGF), Brain-Derived Neurotrophic Factor (BDNF), Glial Cell Line-Derived Neurotrophic Factor (GDNF), Monocyte Chemoattractant Protein 2 (MCP_2), Lymphotoxin-like Inducible Protein (LIGHT), Platelet-Derived Growth Factor BB (PDGF_BB), Hepatocyte Growth Factor (HGF) and Thrombopoietin (TPO), Interleukin-1 alpha (IL_1a), Eotaxin-3 (Eotaxin_3), Transforming Growth Factor Beta 1 (TGF_b1), Growth-Regulated Oncogene Alpha (GRO_a), Interleukin-15 (IL_15), Neurotrophin 4 (NT_4), Insulin-Like Growth Factor I (IGF _I), Tumor Necrosis Factor Beta (TNF_b), Interleukin-1 beta (IL_1_b), Interleukin-5 (IL_5), Glial Cell Line-Derived Neurotrophic Factor (GDNF) and Interleukin-7 (!L_7), Stem Cell Factor (SCF), Fractalkine (CX3CL1), Fibroblast Growth Factor 9 (FGF_9), Macrophage-Derived Chemokine (MDC), Monocyte Chemoattractant Protein 4 (MCP_4), Fibroblast Growth Factor 6 (FGF_6), lnterleukin-7 (IL_7), lnterleukin-5 (IL_5), Leukemia Inhibitory Factor (LIF), and Oncostatin M (Oncostatin_M), Interleukin-12 p70 (IL_12_p70), Eotaxin (Eotaxin), Insulin-Like Growth Factor Binding Protein 4 (IGFBP_4) and Granulocyte-Macrophage Colony-Stimulating Factor (GM_CSF).. Preferably said immunoassay comprises a solid phase carrier comprising a capture and a detection means for each cytokine to be detected as part of the cytokine signature. If the use is for the detection of CFS, the cytokine signature to be detected is one described herein as the basis of the method for aiding in diagnosing PCS. If the use is for distinguishing PCS and PCS with severe ME/CFS, the cytokine signature to be detected is one described herein as the basis of the method for distinguishing between PCS and PCS with severe ME/CFS. Preferably such immunoassay is part of a kit comprising a positive control and/or a negative control. The positive control may comprise antibodies eliciting the release of a cytokine signature, for example a blood sample from a subject suffering from the disease to be diagnosed or one of the diseases to be distinguished, preferably both diseases. The negative control may lack antibodies eliciting the release of a cytokine signature, for example a blood sample from a heathy donor. Another positive control may comprise one or more, preferably all cytokines which are elevated as part of the cytokine signature. Another positive control may comprise one or more, preferably all cytokines of the cytokine signature.

Preferably the immunoassay is an antibody array comprising a capture antibody and a detection antibody for each cytokine. The development and protocols for carrying out such assays are described in the state of the art, for example in Chiswick, E.L., et al., Detection and Quantification of Cytokines and Other Biomarkers. In: Ashman, R. (eds) Leucocytes. Methods in Molecular Biology, vol 844. Humana Press. 2012. https://doi.org/10.1007/978-1-61779-527-5_2. An antibody array based on fluorescence detection (e.g. RayBiotech, Norcross, GA, USA, #AAH-CYT-G5-8) is a preferred option.

The sample needs to comprise an amount of biologically antibodies, preferably autoantibodies, preferably enriched antibodies, more preferably enriched autoantibodies sufficient to elicit a detectable cytokine release from the cell. The collection and preservation of samples comprising such antibodies is described in the state of the art, for example in Wild, The Immunoassay Handbook, 13th edition, Elsevier 2005, in particular chapter 41.

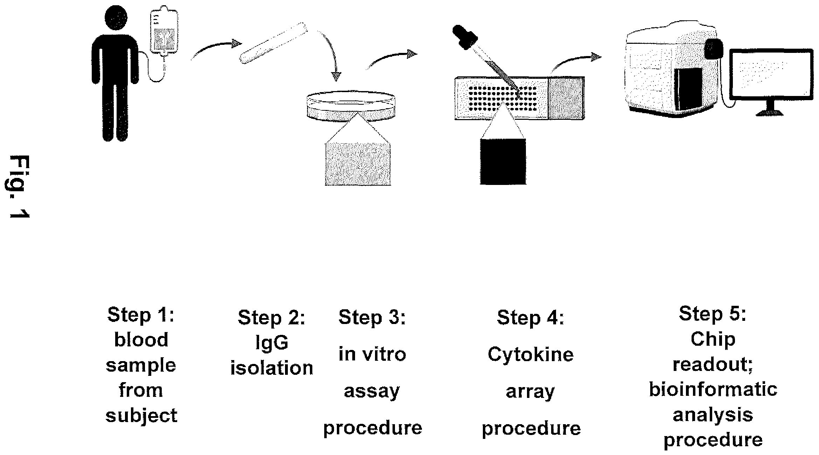

**Fig. 1** illustrates how the methods and uses according to the invention are typically practiced. A blood sample from a subject is obtained (step 1), followed by isolation of IgG antibodies from the sample (step 2). Subsequently, the antibodies are contacted with the U937 cells (step 3) and the cytokine signature is detected (step 4) by carrying out a fluorescence detection assay (step 4), analyzing the chip read out and data analysis using bioinformatic methods (step 5)

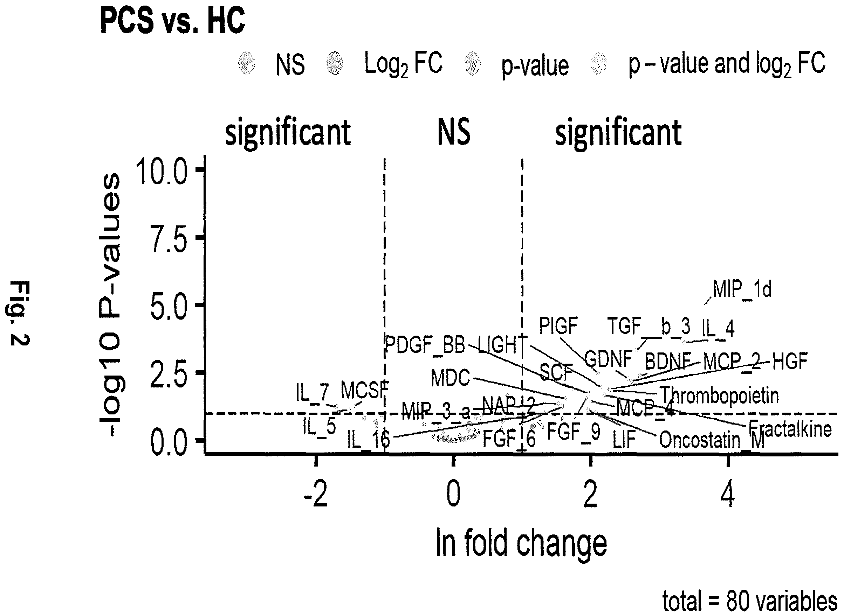

**Fig. 2** shows a volcano plot depicting the significant differences in cytokine expression patterns among two groups, patients suffering from PCS and healthy controls. The plot was derived from batch-corrected data, utilizing linear models to identify significant differences in expression. The plot illustrates the relationship between the log10(p values) and Log2 Fold Change for each group comparison. Points above the horizontal line represent cytokines with significant changes in expression (p<0.05), with the vertical axis indicating the magnitude of the statistical significance and the horizontal insights into the distinct cytokine expression profiles associated with the respective condition.

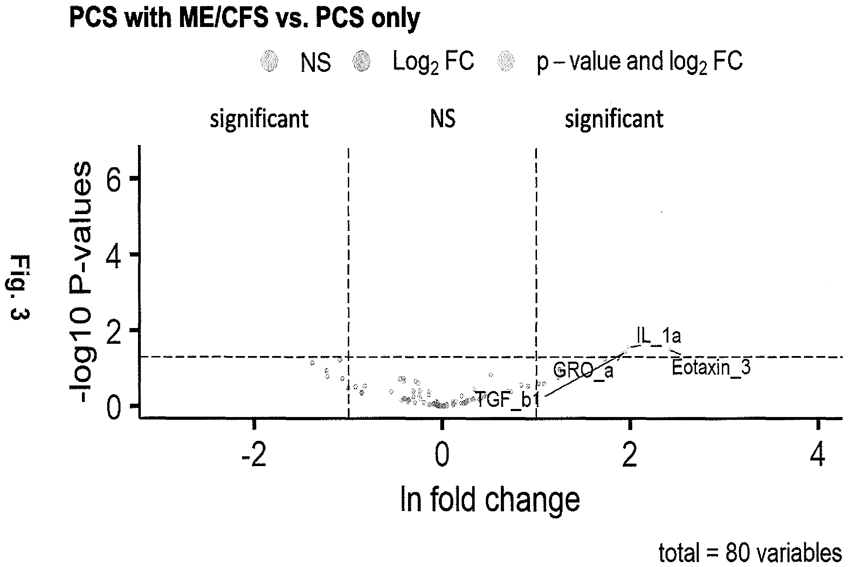

**Fig. 3** shows a volcano plot depicting the significant differences in cytokine expression patterns among two groups, patients suffering from PCS with ME/CFS and patients with PCS only. The plot was derived from batch-corrected data, utilizing linear models to identify significant differences in expression. The plot illustrates the relationship between the log10(p values) and Log2 Fold Change for each group comparison. Points above the horizontal line represent cytokines with significant changes in expression (p<0.05), with the vertical axis indicating the magnitude of the statistical significance and the horizontal insights into the distinct cytokine expression profiles associated with the respective condition.

The present invention is further illustrated by the following non-limiting examples from which further features, embodiments, aspects and advantages of the present invention may be taken. All references cited throughout this application are incorporated by reference.

### Example 1:

### 1. Culturing and preparation of U937 cells

Human monocytic cell line U937 (Sundstrom C, Nilsson K. Establishment and characterization of a human histiocytic lymphoma cell line (U-937). Int. J. Cancer 17: 565-577, 1976. PubMed: 178611) was obtained from the American Type Culture Collection (U937 ATCC CRL-1593.2).

Cells were cultured in RPMI 1640 medium (B&S, #F1415) comprising 10% heat-deactivated fetal calf serum (B&S #FCS-ULE1415) as well as 100 U/ml penicillin and 100 µg/ml streptomycin (BioWest #L0022). Cells were incubated in culture flasks under sterile conditions at 37 °C in the presence of 5% carbon dioxide.

For the following experiments, cells were seeded at a density of 2 × 10^6 cells/ml one hour before stimulation on 24 well plates (Sarstedt, #83.3922500).

### 2. Isolating human IgG

IgG was isolated from patient serum using affinity chromatography. IgG purification was carried out at room temperature, and serum samples as well as the eluted IgG were kept on blue ice. Serum samples from patients were diluted four-fold in binding buffer (20 mM Na₂HPO₄ and 20 mM NaH₂PO₄ at pH 7) and centrifuged for 10 min at 4 °C and 10000 x g. Using a syringe and needle, the serum was aspirated from underneath the lipid layer and transferred to a new tube on ice.

HiTrap protein G Sepharose columns (Cytiva, Sweden, #17040401) were equilibrated in binding buffer. Diluted patient sera were loaded onto the columns and unbound molecules were washed out with binding buffer. Any IgG bound to the column was eluted using 100 mM Glycine at pH 2.7 and immediately upon collection neutralized using 1 M TRIS buffer. The column was again equilibrated with binding buffer for re-use. The neutralized eluates were diluted with sterile PBS (BioWest, #L0615) to 15 ml and passed through centrifugal filter units with 10 kD membrane pore size at 3000 x g at 4°C (Amicon, #UFC901024). The flowthrough was discarded, the retentate was replenished once more to 15 ml with sterile cold PBS and centrifuged again until the retentate's volume was reduced to 500 µl. IgG concentration was assessed with Bradford's reagent (BioRad, #5000006) according to the manufacturer's instructions using the Bovine Gammaglobulin Standard (BioRad, #500-0005) and measured with Eppendorf's Bio-Photometer.

### 3. Stimulation of U937 cells by IgG obtained from patients or healthy donors

Cultivated and seeded U937 cells were stimulated for 24 hours in RPMI 1640 (Thermo Fischer Scientific) by addition to a final concentration of a mg ml⁻¹ of IgG was purified from the following defined groups: a) patients with PCS, b) patients suffering from PCS with ME/CFS, and c) healthy subjects. Following stimulation procedures, cell suspensions were centrifuged, and the supernatant was preserved at -20 °C for later analysis.

The clinical status of participants was thoroughly documented, encompassing the intensity and presence of key symptoms such as fatigue, muscle pain, immune responses, cognitive disruptions, and signs of autonomic dysfunction, using appropriate questionnaires. The COMPASS 31 was employed for patient characterization.

For PCS patients, the criteria included a confirmed mild to moderate COVID-19 diagnosis, established through PCR or serology, and persistent moderate to severe fatigue and exertion intolerance post SARS-CoV-2 infection. Exclusion criteria involved significant cardiac, respiratory, neurological, or psychiatric comorbidities, preexisting fatigue, or any organ dysfunction evidence. PCS diagnosis was made at least six months post COVID-19, with retrospectively confirmed no later than two months following blood sampling.

ME/CFS diagnosis adhered to the 2003 Canadian Consensus Criteria, (Bruce M. Carruthers, Anil Kumar Jain, Kenny L. De Meirleir, Daniel L. Peterson, Nancy G. Klimas, A. Martin Lerner, Alison C. Bested, Pierre Flor-Henry, Pradip Joshi, A. C. Peter Powles, Jeffrey A. Sherkey & Marjorie I. van de Sande (2003) Myalgic Encephalomyelitis/Chronic Fatigue Syndrome, Journal Of Chronic Fatigue Syndrome, 11:1, 7-115, DOI: 10.1300/J092v11n01_02) (CCC), as recommended by EUROMENE for research purposes. Patients meeting the CCC standards exhibited continuous fatigue for a minimum of six months, post-exertional malaise (PEM), sleep disturbances, pain, a minimum of two neurological or cognitive symptoms, and at least one symptom from two of the categories: autonomic, neuroendocrine, or immune manifestations. Other potential causes of fatigue, such as certain medical or neurological diseases, being ruled out through extensive laboratory tests. Additionally, important information regarding the type of infection that acted as a trigger for the disease, as well as the time of onset, is documented under EU General Data Protection Regulation (GDPR), particularly Articles 6 and 9 and German Patient Rights Act (Patientenrechtegesetz), providing a framework for patient rights including data privacy.

All PCS (eight patients) and PCS with severe ME/CFS (seven patients) diagnoses were carried out by experienced clinicians who were certified specialists in the field of internal medicine and/or rheumatology. Sixteen samples from healthy donors were considered.

Cell-free supernatants were transferred onto cytokine antibody array glass chips (chamber slides) carrying specific, immobilized antibodies against a total of 80 selected cytokines as well as several positive and negative controls (RayBiotech, Norcross, GA, USA, #AAH-CYT-G5-8). This assay is recognized for its high sensitivity and specificity, as briefly described in the available literature and the manufacturer's claims. The sensitivity of the assay is notable, with the capability to detect cytokine concentrations in the range of picograms per milliliter (pg/mL) to nanograms per milliliter (ng/mL). This level of sensitivity is vital for accurately measuring low concentrations of cytokines in our samples. Additionally, the assay's high specificity minimizes the occurrence of false positives and negatives, thereby ensuring reliable detection of the target cytokines. The manufacturer's instructions were followed. Briefly, a glass chip comprising multiple capture antibodies is contacted with the sample, incubated and then contacted with a cocktail of biotinylated detection antibodies. Subsequently, streptavidin labelled with a fluorescent dye is added, followed by detection of the label using a laser scanner

The protocol was carried out according to the manufacturer's instructions: Briefly, non-specific binding sites on the chamber slides were blocked with blocking buffer, followed by incubation with the supernatants of the stimulated cells. After multiple washes, the chamber slides were incubated with a cocktail of specific biotinylated secondary antibodies. Following repeated washing, the streptavidin-fluorochrome reagent HiLyte Plus^{™} Fluor 555 was added to the chamber slides and incubated in the dark. After a final extensive wash, the chamber slides were disassembled, dried, and laser-scanned at 532 nm (Agilent SureScan Microarray Scanner G2600D, Agilent Technologies, Santa Clara, USA). The background was subtracted, and the signals were normalized to the positive control signals prior to data analysis (Scan Control Software ver9. 1, Agilent Technologies, Santa Clara, USA).

The data were 'batch-corrected' using the array manufacturer's batch correction file (https://www.raybiotech.com/tools/array-analysis-tool/). Further processing was conducted in R (version 4.2.1) by using RStudio (version 7.1). Limma-Batch Correction: The data were loaded into R and prepared for an additional batch correction. This correction is necessary to remove non-biological variances that may arise from different experimental batches. The limma package function in R is used to correct these batch effects. This is done by modeling and adjusting batch variables in the linear model equation of the limma analysis. L.imma Analysis for Group Comparisons (healthy subjects, PCS, PCS with with severe ME/CFS): A differential expression analysis is conducted with the limma package. This involves comparing the groups healthy subjects, PCS with with severe ME/CFS and PCS (only post-Covid not for filling compass 31 criteria, or within the defined 6-month range after Covid-19 infection). The focus was on identifying significant differences in the expression patterns of these groups. This is achieved by applying linear models to the batch-corrected data. After identifying the significant differences, volcano plots were created to visually represent them. In these plots, - log10(p-values) were plotted against the Log2-Fold-Change to highlight the significant changes (with a threshold of p = 0.05) **(****Fig. 2** and **Fig. 3****)**

First, it was determined that a cytokine signature comprising those listed in Table1 and Tables 2 is statistically relevant for determining whether or not a subject suffers from PCS, including PCS with severe ME/CFS, whereas other cytokines were found to be irrelevant. These cytokines were determined by statistical significance by the shown limma analyses **(****Fig. 2****)** combined with expert-knowledge in relevance to medical and biological function of cytokines, cytokines without significant p-value, but moderate fold-change between groups were chosen with expert knowledge in order to ensure medical relevance of the testing procedure. Linear Discriminant Analysis (LDA) was performed on the selected cytokines to classify discriminate, respectively healthy participants from all PCS and discriminating PCS with ME/CFS against PCS without ME/CFS. The LDA model was trained using the Ida-function from the MASS package in R. Predicted posterior probabilities for group classification were obtained, and the optimal threshold for discrimination was determined using the roc function from the pROC package. The threshold, sensitivity, and specificity were calculated using the coords function, selecting the threshold that maximized the sum of sensitivity and specificity. Model performance was assessed using the Receiver Operating Characteristic (ROC) curve. A core cytokine signature (Table 1) and an expanded cytokine signature (Table 2) could be defined that may be used to aid in the diagnosis of PCS.

Table 1 shows the core cytokine signature resulting from the comparison of healthy subjects to the entirety of subjects suffering from PCS and PCS with severe ME/CFS. A sensitivity of 76% and a specificity of 74%) was determined.

**Table 1 Log2 fluorescence cut off values resemble log2 of median fluorescent intensity = log2[MFI] of cy3 or "green" excitation frequency = 532 nm. The respective cytokine level as determined using the RayBiotech, Norcross, GA, USA, #AAH-CYT-G5-8 as described in the examples. ("<" means that a level of the respective cytokine lower than the indicated cut off value indicates an increased likelihood that a sample examined is from a subject suffer from PCS (i.e. threshold is a maximum threshold). ">" means that a level of the respective cytokine higher than the indicated cut off value indicates an increased likelihood that a sample examined is from a subject suffer from PCS (i.e. threshold is a minimum threshold).**

| **Cytokines** | **Threshold** | |
|---|---|---|
| Macrophage Inflammatory Protein 1-delta (MiP_1d) | 8.65 | > |
| lnterleukin-4 (IL_4) | 7.4 | > |
| Transforming Growth Factor Beta 3 (TGF_b_3) | 8.85 | > |
| Placental Growth Factor (PIGF) | 8.27 | > |
| Brain-Derived Neurotrophic Factor (BDNF) | 9.83 | > |
| Glial Cell Line-Derived Neurotrophic Factor (GDNF) | 10.35 | > |
| Monocyte Chemoattractant Protein 2 (MCP_2) | 5.31 | > |
| Lymphotoxin-like Inducible Protein (LIGHT) | 8.72 | > |
| Platelet-Derived Growth Factor BB (PDGF_BB) | 8.06 | > |
| Hepatocyte Growth Factor (HGF) | 6.99 | > |
| Thrombopoietin (TPO) | 11.73 | > |

Table 2 shows an expanded cytokine signature resulting from the comparison of healthy subjects to the entirety of subjects suffering from PCS and PCS with severe ME/CFS. A sensitivity of 92% and a specificity of 83%) was determined.

**Table 2 Log2 fluorescence cut off values resemble log2 of median fluorescent intensity = log2[MFI] of cy3 or "green" excitation frequency = 532 nm. The respective cytokine level as determined using the RayBiotech, Norcross, GA, USA, #AAH-CYT-G5-8 as described in the examples. ("<" means that a level of the respective cytokine lower than the indicated cut off value indicates an increased likelihood that a sample examined is from a subject suffer from PCS (i.e. threshold is a maximum threshold). ">" means that a level of the respective cytokine higher than the indicated cut off value indicates an increased likelihood that a sample examined is from a subject suffer from PCS (i.e. threshold is a minimum threshold).**

| **Cytokines** | **Threshold** | |
|---|---|---|
| Macrophage Inflammatory Protein 1-delta (MIP_1d) | 8.65 | > |
| lnterleukin-4 (IL_4) | 7.4 | > |
| Transforming Growth Factor Beta 3 (TGF_b_3) | 8.85 | > |
| Placental Growth Factor (PIGF) | 8.27 | > |
| Brain-Derived Neurotrophic Factor (BDNF) | 9.83 | > |
| Glial Cell Line-Derived Neurotrophic Factor (GDNF) | 10.35 | > |
| Monocyte Chemoattractant Protein 2 (MCP_2) | 5.31 | > |
| Lymphotoxin-like Inducible Protein (LIGHT) | 8.72 | > |
| Platelet-Derived Growth Factor BB (PDGF_BB) | 8.06 | > |
| Hepatocyte Growth Factor (HGF) | 6.99 | > |
| Thrombopoietin (TPO) | 11.73 | > |
| Stem Cell Factor (SCF) | 10.19 | > |
| Fractalkine (CX3CL1) | 11.42 | > |
| Fibroblast Growth Factor 9 (FGF_9) | 8.73 | > |
| Macrophage-Derived Chemokine (MDC) | 7.98 | > |
| Monocyte Chemoattractant Protein 4 (MCP_4) | 8.16 | > |
| Fibroblast Growth Factor 6 (FGF_6) | 7.53 | > |
| lnterleukin-7 (IL_7) | 10.68 | < |
| lnterleukin-5 (IL_5) | 8.61 | < |
| Leukemia Inhibitory Factor (LIF) | 8.65 | > |
| Oncostatin M (Oncostatin_M) | 9.68 | > |

Second, it was determined that a cytokine core signature comprising those listed in Table 3 and Tables 4 is statistically relevant for distinguishing whether or not a subject suffers from PCS or PCS with severe ME/CFS, whereas other cytokines were found to be irrelevant. Significant cytokines were chosen by significant p-value and at least a moderate foldchange by expert-knowledge and tested for importance by Ida. A core cytokine signature (Table 3) and an expanded cytokine signature (Table 4) could be defined that may be used to aid in distinguishing between PCS and PCS with severe ME/CFS.

Table 3 shows the core signature resulting from the comparison of subjects suffering from PCS and subjects suffering from PCS with severe ME/CFS. A sensitivity of 85% and a specificity of 67%) was determined.

**Table 3 Log2 fluorescence cut off values resemble log2 of median fluorescent intensity = log2[MFI] of cy3 or "green" excitation frequency = 532 nm. The respective cytokine level as determined using the RayBiotech, Norcross, GA, USA, #AAH-CYT-G5-8 as described in the examples. "<" means that a level of the respective cytokine lower than the indicated cut off value indicates an increased likelihood that a sample examined is from a subject suffering from PCS with ME/CFS rather than from a subject suffering from PCS without ME/CFS (i.e. threshold is a maximum threshold). ">" means that a level of the respective cytokine higher than the indicated threshold value indicates an increased likelihood that a sample examined is from a subject suffering from PCS with ME/CFS rather than from a subject suffering from PCS without ME/CFS (i.e. threshold is a minimum threshold).**

| **Cytokines** | **Threshold** | |
|---|---|---|
| lnterleukin-1 alpha (IL_1a) | 8.68 | > |
| Eotaxin-3 (Eotaxin_3) | 8.31 | > |
| Transforming Growth Factor Beta 1 (TGF_b1) | 9.17 | > |
| Growth-Regulated Oncogene Alpha (GRO_a) | 9.81 | > |
| Interleukin-15 (IL_15) | 8.7 | > |
| Neurotrophin-4 (NT_4) | 8.28 | < |
| Insulin-Like Growth Factor I (IGF_I) | 8.7 | < |
| Tumor Necrosis Factor Beta (TNF_b) | 13.68 | > |
| Interleukin-1 beta (IL_1_b) | 11.2 | < |
| lnterleukin-5 (IL_5) | 8.6 | > |
| Glial Cell Line-Derived Neurotrophic Factor (GDNF) | 14.43 | < |
| Interleukin-7 (IL_7) | 8.26 | > |

Table 4 shows an expanded cytokine signature resulting from the comparison of subjects suffering from PCS and subjects suffering from PCS with severe ME/CFS. A sensitivity of 92% and a specificity of 83%) was determined.

**Table 4 Log2 fluorescence cut off values resemble log2 of median fluorescent intensity = log2[MFI] of cy3 or "green" excitation frequency = 532 nm. The respective cytokine level as determined using the RayBiotech, Norcross, GA, USA, #AAH-CYT-G5-8 as described in the examples. "<" means that a level of the respective cytokine lower than the indicated threshold value indicates an increased likelihood that a sample examined is from a subject suffering from PCS with ME/CFS rather than from a subject suffering from PCS without ME/CFS (i.e. threshold is a maximum threshold). ">" means that a level of the respective cytokine higher than the indicated threshold value indicates an increased likelihood that a sample examined is from a subject suffering from PCS with ME/CFS rather than from a subject suffering from PCS without ME/CFS (i.e. threshold is a minimum threshold).**

| **Cytokines** | **Threshold** | |
|---|---|---|
| Interleukin-1 alpha (IL_1a) | 8.68 | > |
| Eotaxin-3 (Eotaxin_3) | 8.31 | > |
| Transforming Growth Factor Beta 1 (TGF_b1) | 9.17 | > |
| Growth-Regulated Oncogene Alpha (GRO_a) | 9.81 | > |
| Interleukin-15 (IL_15) | 8.7 | > |
| Neurotrophin-4 (NT_4) | 8.28 | < |
| Insulin-Like Growth Factor I (IGF_I) | 8.7 | < |
| Tumor Necrosis Factor Beta (TNF_b) | 13.68 | > |
| Interleukin-1 beta (IL_1_b) | 11.2 | < |
| lnterleukin-5 (IL_5) | 8.6 | > |
| Glial Cell Line-Derived Neurotrophic Factor (GDNF) | 14.43 | < |
| Interleukin-7 (IL_7) | 8.26 | > |
| Interleukin-12 p70 (IL_12_p70) | 9.89 | < |
| Eotaxin (Eotaxin) | 9.04 | < |
| Insulin-Like Growth Factor Binding Protein 4 (IGFBP_4) | 13.27 | < |
| Granulocyte-Macrophage Colony-Stimulating Factor (GM_CSF) | 8.89 | > |
| Monocyte Chemoattractant Protein 4 (MCP_4) | 7.85 | > |

## Claims

1. A method for aiding in the diagnosis of Post Covid Syndrome (PCS) in a mammalian subject comprising the steps
a) contacting ex *vivo* a mammalian cell line capable of secreting cytokines with a set of antibodies from a blood sample of the mammalian subject in a liquid medium,
b) detecting a cytokine signature present in the liquid medium following step a), wherein the cytokine signature detected comprises Macrophage Inflammatory Protein 1-delta (MIP_1d), lnterleukin-4 (IL_4), Transforming Growth Factor Beta 3 (TGF_b_3), Placental Growth Factor (PIGF), Brain-Derived Neurotrophic Factor (BDNF), Glial Cell Line-Derived Neurotrophic Factor (GDNF), Monocyte Chemoattractant Protein 2 (MCP_2), Lymphotoxin-like Inducible Protein (LIGHT), Platelet-Derived Growth Factor BB (PDGF_BB), Hepatocyte Growth Factor (HGF) and Thrombopoietin (TPO).
and the mammalian cell line is U937.

2. A method for aiding in distinguishing between PCS and PCS with severe myalgic encephalomyelitis/chronic fatigue syndrome (ME/CFS) in a mammalian subject comprising the steps
a) contacting *ex vivo* a mammalian cell line capable of secreting cytokines with a set of antibodies from a blood sample of the mammalian subject in a liquid medium,
b) detecting a cytokine signature present in the liquid medium following step a), wherein the cytokine signature detected comprises Interleukin-1 alpha (IL_1a), Eotaxin-3 (Eotaxin_3), Transforming Growth Factor Beta 1 (TGF_b1), Growth-Regulated Oncogene Alpha (GRO_a), Interleukin-15 (IL_15), Neurotrophin 4 (NT_4), Insulin-Like Growth Factor I (lGF_I), Tumor Necrosis Factor Beta (TNF_b), Interleukin-1 beta (IL_1_b), Interleukin-5 (IL_5), Glial Cell Line-Derived Neurotrophic Factor (GDNF) and Interleukin-7 (IL_7)
and the mammalian cell line is U937.

3. The method according to any one of claims 1 to 2, wherein the set of antibodies comprises isolated and/or concentrated antibodies from the sample from the subject.

4. The method according to any of claims 1 to 3, wherein the set of antibodies comprises IgG, IgM and IgA class antibodies.

5. The method according to any of claims 1 to 4, wherein the set of antibodies comprises IgG class antibodies, preferably enriched relative to other classes of antibodies.

6. The method according to step 5, wherein the set of IgG class antibodies comprises IgG class antibodies isolated from the blood sample using affinity chromatography.

7. The method according to any of claims 1 to 6, comprising the step separating the cell from the liquid medium following step b).

8. The method according to any of claims 1 to 7, wherein the liquid medium is frozen after step a).

9. he method according to any of claims 1 to 8, wherein a method from the group comprising ELISA, flow cytometry and antibody microarray is used to detect the cytokine signature.

10. The method according to any of steps 1 to 9, wherein the subject is selected from the group comprising a rodent, a dog, a bird, a cat, a horse, a sheep, a rabbit, a goat and a primate, and preferably is a human subject.

11. The method according to any of claims 1 to 10, wherein at least two sets of antibodies are used in separate runs, preferably one from a healthy subject and one from a subject suspected of having a disease.

12. The method according to any of claims 1 to 11, wherein the sample is a blood sample selected from the group comprising serum, whole blood, plasma and capillary blood.

13. The method according to any of claims 1 to 12, wherein the cell is an isolated cell.

14. The method according to any of claims 1 and 3 to 13, wherein the cytokine signature comprises in addition the group comprising Stem Cell Factor (SCF), Fractalkine (CX3CL1), Fibroblast Growth Factor 9 (FGF_9), Macrophage-Derived Chemokine (MDC), Monocyte Chemoattractant Protein 4 (MCP_4), Fibroblast Growth Factor 6 (FGF_6), Interleukin-7 (IL_7), Interleukin-5 (IL_5), Leukemia Inhibitory Factor (LIF), Oncostatin M and (Oncostatin_M).

15. The method according to any of claims 2 to 13, wherein the cytokine signature comprises in addition the group comprising Interleukin-12 p70 (IL_12_p70), Eotaxin (Eotaxin), Insulin-Like Growth Factor Binding Protein 4 (IGFBP_4), Granulocyte-Macrophage Colony-Stimulating Factor (GM_CSF) and Monocyte Chemoattractant Protein 4 (MCP_4).
